Europäisches Patentamt

European Patent Office

Office européen des brevets

(11) Veröffentlichungsnummer: **0 068 189**
**B1**

## (12) EUROPÄISCHE PATENTSCHRIFT

(45) Veröffentlichungstag der Patentschrift:
03.10.84

(21) Anmeldenummer: 82104996.2

(22) Anmeldetag: 08.06.82

(51) Int. Cl.³: **A 61 L 15/00, C 08 F 220/00**

(54) Vernetzte, in Wasser quellbare Copolymerisate und ihre Verwendung als Absorptionsmittel für wässrige Körperflüssigkeiten wie Urin und andere elektrolythaltige wässrige Flüssigkeiten.

(30) Priorität: 19.06.81 DE 3124008

(43) Veröffentlichungstag der Anmeldung:
05.01.83 Patentblatt 83/1

(45) Bekanntmachung des Hinweises auf die Patenterteilung:
03.10.84 Patentblatt 84/40

(84) Benannte Vertragsstaaten:
BE CH DE FR GB IT LI SE

(56) Entgegenhaltungen:
DE - A - 2 218 100
GB - A - 2 053 937
GB - A - 2 064 556

(73) Patentinhaber: Chemische Fabrik Stockhausen GmbH,
Bäkerpfad 25, D-4150 Krefeld (DE)

(72) Erfinder: Chmelir, Miroslav, Dr. Dipl.-Chem.,
Grönkesdyk 36, D-4150 Krefeld (DE)
Erfinder: Dahmen, Kurt, Dr. Dipl.-Chem., von Veisen
Strasse 6, D-4050 Mönchengladbach (DE)
Erfinder: Türk, Wolfgang, Dr. Dipl.-Chem.,
Sollbrüggenstrasse 80, D-4150 Krefeld (DE)

(74) Vertreter: Klöpsch, Gerald, Dr.-Ing., An Gross St.
Martin 6, D-5000 Köln 1 (DE)

ACTORUM AG

# Beschreibung

Die Erfindung betrifft vernetzte, in Wasser quellbare Copolymerisate von 2-Acrylamido-2-methylpropansulfonsäure mit Acrylsäure, Methacrylsäure, Acrylamid und/oder Vinylpyrrolidon, die mittels eines mindestens bifunktionellen Vernetzers vernetzt sind, sowie die Verwendung dieser Copolymerisate als Absorptionsmittel für wässerige Körperflüssigkeiten, wie Urin und andere elektrolythaltige wässerige Lösungen, insbesondere ihre Verwendung in absorbierenden Wegwerferzeugnissen für medizinische Zwecke sowie für Hygieneartikel.

In den letzten Jahren wurde eine Anzahl verschiedener Polymerisate entwickelt, die hohes Absorptionsvermögen für Wasser und Körperflüssigkeiten aufweisen. Die meisten, teilweise vernetzten Produkte wurden auf Stärkebasis, wie z.B. Stärke/Acrylnitril-Pfropfpolymerisate (US-PS Nrn. 3997484, 3661815, 4155888, 3935099), gelatinisierte Stärkederivate (DE-OS Nr. 2702781) oder auf Cellulosebasis, wie Derivate von Alkyl- oder Hydroxyalkylcellulose (JA-PS Nr. 77/125481), Carboxmethylcellulose (BE-PS Nr. 862130, GB-PS Nr. 1519949) und auf Polysaccharidbasis (DE-OS Nr. 2650377) hergestellt. Zu den vollsynthetischen, in zahlreichen Patenten beschriebenen Absorptionsmitteln gehören vernetzte Polymere und Copolymere auf Acryl- oder Methacrylsäurebasis (DE-OS Nrn. 2429236, 2614662, US-PS Nrn. 4018951, 3926891, 4066583, 4062817, DE-OS Nrn. 2712043, 2653135, 2650377, 2813634) oder Maleinsäurederivate (US-PS Nr. 4041228).

Alle diese Produkte sind praktisch wasserunlöslich, absorbieren das Vielfache ihres Gewichts an Wasser, aber deutlich weniger an wässerigen, elektrolythaltigen Lösungen sowie an Urin.

Es wurde nun gefunden, dass ein vernetztes Copolymerisat der 2-Acrylamido-2-methylpropansulfonsäure deutlich höhere Mengen an elektrolythaltiger Lösung absorbieren kann als die bisherigen, nach dem Stand der Technik hergestellten Polymerisate oder Copolymerisate.

Gegenstand der Erfindung sind vernetzte, in Wasser quellbare Copolmerisate, bestehend aus

A) 0,5 bis 75, vorzugsweise 5 bis 30 Gew.-% Grundeinheiten, die sich von 2-Acrylamido-2-methylpropansulfonsäure oder deren Alkali- und/oder Ammoniumsalzen ableiten,

B) 23 bis 99,49, vorzugsweise 70 bis 95 Gew.-% Grundeinheiten, die sich von Acrylsäure und/oder Methacrylsäure oder deren Alkali- und/oder Ammoniumsalzen und/oder Acrylamid und/oder Vinylpyrrolidon ableiten, und

C) 0,01 bis 2,0 Gew.-% an Grundeinheiten, die sich von einem mindestens bifunktionellen Vernetzer ableiten.

Die erfindungsgemässen Copolymerisate werden durch die Copolymerisation von 2-Acrylamido-2-methylpropansulfonsäure mit anderen Monomeren, wie Acrylsäure, Methacrylsäure, Acrylamid, Vinylpyrrolidon in Gegenwart eines mindestens bifunktionellen Vernetzers, wie z.B.

Methylenbisacrylamid, Triallylphosphat, Trimethylolpropandiallylether, Tetraallyloxyethan u.a., hergestellt. Die Copolymerisation kann nach bekannten Verfahren durchgeführt werden.

Gegenstand der Erfindung ist ferner ein Absorptionsmittel für Körperflüssigkeiten, wie Urin und andere elektrolythaltige wässerige Flüssigkeiten auf der Basis der erfindungsgemässen Copolymerisate.

Die erfindungsgemässen Absorptionsmittel eignen sich besonders für den Einsatz in Saugkörpern wie Babywindeln und Inkontinenzartikeln. Wegen ihrer guten Rieselfähigkeit lassen sich die Absorptionsmittel auf entsprechenden Eintragungsaggregaten gut verarbeiten. Als Trägermaterial kommen Textilgebilde auf Gewebe- oder Vliesbasis sowie Papiererzegnisse in Frage, auf denen die Absorptionsmittel durch geeignete Massnahmen fixiert werden.

Die erfindungsgemässen Absorptionsmittel zeigen auch bei stark elektrolythaltigem Wasser (z.B. Meerwasser oder Brackwasser) gute Absorptionswerte und sind insbesondere im Zyklusversuch (wiederholte Wasseraufnahme nach Trocknung des aufgequollenen Absorptionsmittels) den Produkten des Standes der Technik, wie z.B. vernetzten Acrylamid/Acrylsäure-Copolymerisaten überraschend überlegen.

Für die Beschleunigung der Flüssigkeitsaufnahme können den erfindungsgemässen Absorptionsmitteln noch andere hydrophile Substanzen, wie z.B. auf pyrolytischem Wege oder durch Fällung aus Wasserglaslösungen gewonnene feinverteilte Kieselsäure, in einer Menge von 0,1 bis 10, vorzugsweise 0,2 bis 5 Gew.-%, zugesetzt werden. Es können weiterhin Riechstoffe, Deodorantien, Desinfektionsmittel, Bindemittel oder sonstige Stoffe, die die Absorptionseigenschaften des Absorptionsmaterials nicht beeinflussen, zugemischt werden.

Zur Demonstration der Gebrauchseigenschaften wird die Aufnahme von Modellurin nach dem *Demand-Absorbency*-Test (W.F. Schlauch, „Vortrag Index", 1978, Amsterdam) durchgeführt:

Das Messgerät besteht aus einer Bürette, die mit der Modellurinlösung (2,0% Harnstoff, 0,9% NaCl, 0,1% $MgSO_4$ und 0,06% $CaCl_2$ aufgelöst in destilliertem Wasser) gefüllt ist, und einem Probetisch, der mit einer an die Messbürette angeschlossenen Öffnung für den Modellurinlösungsaustritt vorgesehen ist. Auf den mit einem dünnen Vlies (10 × 13,5 cm) bedeckten Probetisch wird 0,5 g des erfindungsgemässen Produkts in Form einer kreisrunden Fläche von 4,5 cm Durchmesser, zentrisch über dem Flüssigkeitsaustritt, gleichmässig aufgestreut. Durch Schliessen des Schlauches und leichte Druckgebung wird der Kontakt der Modellurinlösung mit dem Pulverprodukt hergestellt, so dass die Modellurinlösung durch das erfindungsgemässe Produkt absorbiert werden kann. Die absorbierte Menge der Modellurinlösung wird nach 20 min. abgelesen. Anschliessend wurde die Retention durch Belastung des gequollenen Geles mit einem Gewicht von 10 g/cm² ermittelt. Die Zeit der Belastung betrug 5 min.

Die folgenden Beispiele sollen die Erfindung erläutern:

*Beispiel 1*

In einem Polymerisationsgefäss wurden 326 g Acrylsäure und 49,2 g Acrylamidomethylpropansulfonsäure in 965 g destilliertem Wasser gelöst und mit 130 g Natriumhydrogencarbonat ein pH = 4,0 eingestellt. Danach wurde 0,37 g N,N'-Methylenbisacrylamid zugegeben und mit 0,6 g Azobisamidinpropandihydrochlorid unter Einwirkung von UV-Licht katalysiert. Die Reaktion verlief adiabatisch, wobei die Temperatur von 15 bis zu 75°C gestiegen ist. Das entstandene Polymergel wurde zerkleinert, getrocknet und gemahlen. Das vernetzte Copolymere absorbiert 53 ml Modellurin ohne Belastung und 37 ml Modellurin unter Belastung (10 g/cm²) pro 1 g Produkt.

*Beispiel 2*

In einem Polymerisationsgefäss wurden 284 g Acrylsäure und 90,7 g Acrylamidomethylpropansulfonsäure in 965 g destilliertem Wasser gelöst und mit 130 g Natriumhydrogencarbonat ein pH = 4,5 eingestellt. Danach wurde 0,35 g Tetraallyloxyethan zugegeben und mit 0,73 g Kaliumpersulfat, 1,34 g Natriumpyrosulfat und 0,06 g Eisen(II)-gluconat, gelöst in 120 ml Wasser, katalysiert. Die Reaktion verlief adiabatisch, wobei die Temperatur von 15 bis zu 70°C gestiegen ist. Das entstandene Polymergel wurde zerkleinert, getrocknet und gemahlen. Das vernetzte Copolymere absorbiert 53 ml Modellurin ohne Belastung und 38 ml Modellurin unter Belastung (10 g/cm²) pro 1 g Produkt, sowie 64 ml/g (6400%) isotonische Lösung (0,9%ige wässerige NaCl-Lösung).

*Beispiel 3*

In einem Polymerisationsgefäss wurden 218 g Acrylsäure und 157 g Acrylamidomethylpropansulfonsäure in 965 g destilliertem Wasser gelöst und mit 134 g Natriumhydrogencarbonat ein pH = 4,3 eingestellt. Danach wurde 0,37 g Trimethylolpropandiallylether zugegeben und mit 0,6 g Azobisamidinpropandihydrochlorid unter Einwirkung von UV-Licht katalysiert. Die Reaktion verlief adiabatisch, wobei die Temperatur von 15 bis zu 76°C gestiegen ist. Das entstandene Polymergel wurde zerkleinert, getrocknet und gemahlen. Das vernetzte Copolymere absorbiert 52,5 ml Modellurin ohne Belastung und 41 ml Modellurin unter Belastung (10 g/cm²) pro 1 g Produkt sowie 61 ml/g (6100%) isotonische Lösung (0,9%ige wässerige NaCl-Lösung).

*Beispiel 4*

In einem Polymerisationsgefäss wurden 128 g Acrylsäure und 246 g Acrylamidomethylpropansulfonsäure in 965 g destilliertem Wasser gelöst und mit 144 g Ammoniumhydrogencarbonat ein pH = 4,1 eingestellt. Danach wurde 0,37 g N,N'-Methylenbisacrylamid zugegeben, auf 50°C erhitzt und mit 1,2 g Azobisamidinpropandihydrochlorid katalysiert. Das entstandene Polymergel wurde zerkleinert, getrocknet, gemahlen und mit 0,5% (bezogen auf das Polymerisat) pyrogener Kieselsäure (Aerosil 200 der Fa. Degussa) vermischt. Das vernetzte Copolymere absorbiert 58 ml Modellurin ohne Belastung und 43 ml Modellurin unter Belastung (10 g/cm²) pro 1 g Produkt.

*Beispiel 5*

In einem Polymerisationsgefäss wurden 222 g Acrylsäure, 111 g Methacrylsäure und 42,1 g Acrylamidomethylpropansulfonsäure in 1050 g destilliertem Wasser gelöst und mit 140 g Natriumhydrogencarbonat teilweise neutralisiert. Danach wurde 0,37 g N,N'-Methylenbisacrylamid zugegeben und mit 0,36 g Azobisamidinpropandihydrochlorid, 0,7 g Kaliumpersulfat und 1,4 g Natriumpyrosulfit katalysiert. Die Reaktion verlief adiabatisch, wobei die Temperatur von 15 bis zu 87°C gestiegen ist. Das entstandene Polymergel wurde zerkleinert, getrocknet und gemahlen. Das vernetzte Copolymere absorbiert 41 ml Modellurin ohne Belastung und 29 ml Modellurin unter Belastung (10 g/cm²) pro 1 mg Produkt.

*Beispiel 6*

In einem Polymerisationsgefäss wurden 325 g Acrylamid und 49,8 g Acrylamidomethylpropansulfonsäure in 1070 g destilliertem Wasser gelöst und mit 26 g Natriumhydrogencarbonat ein pH = 6,8 eingestellt. Danach wurde 0,37 g N,N'-Methylenbisacrylamid zugegeben und mit 0,6 g Azobisamidinpropandihydrochlorid katalysiert. Die Reaktion verlief adiabatisch, wobei die Temperatur von 15 bis zu 82,5°C gestiegen ist. Das entstandene Polymergel wurde zerkleinert, getrocknet und gemahlen. Das vernetzte Copolymere absorbiert 36,5 ml Modellurin ohne Belastung und 20 ml Modellurin unter Belastung (10 g/cm²) pro 1 g Produkt sowie 35 ml/g (3500%) isotonische Lösung (0,9%ige wässerige NaCl-Lösung).

*Beispiel 7*

In einem Polymerisationsgefäss wurden 100 g Vinylpyrrolidon, 230 g Acrylsäure und 42 g Acrylamidomethylpropansulfonsäure in 1000 g destilliertem Wasser gelöst und mit 105 g Natriumhydrogencarbonat teilweise neutralisiert. Danach wurde 0,37 g N,N'-Methylenbisacrylamid zugegeben und mit 0,6 g Azobisamidinpropandihydrochlorid unter Einwirkung von UV-Licht katalysiert. Die Reaktion verlief adiabatisch, wobei die Temperatur von 15 bis 71°C gestiegen ist. Das entstandene Polymergel wurde zerkleinert, getrocknet, gemahlen und mit 0,5% (bezogen auf das Polymerisat) pyrogener Kieselsäure (Aerosil 200 der Fa. Degussa) vermischt. Das vernetzte Copolymere absorbiert 45 ml Modellurin ohne Belastung und 32 ml Modellurin unter Belastung (10 g/cm²) pro 1 g Produkt.

In den folgenden Beispielen wird die Überlegenheit der erfindungsgemässen Absorptionsmittel bei der Absorption von stark elektrolythaltigem Wasser (künstliches Meerwasser) gegenüber bekannten Absorptionsmitteln auf der Basis von vernetzter Polyacrylsäure bzw. einem vernetzten Copolymeren von Acrylamid und Acrylsäure gezeigt.

*Beispiel 8*

In einem Polymerisationsgefäss wurden 332 g Acrylamid und 108 g Acrylamidomethylpropansulfonsäure in 1 000 g Wasser gelöst und mit 36 g Kaliumcarbonat neutralisiert. Danach wurde 1,8 g N,N'-Methylenbisacrylamid zugegeben und mit 0,73 g Azobisamidinpropandihydrochlorid katalysiert. Die Reaktion verlief praktisch adiabatisch, wobei die Temperatur von 20 bis zu 90°C gestiegen ist. Das entstandene Polymergel wurde zerkleinert, getrocknet und gemahlen.

Das Produkt wurde dann mit einem künstlichen Meerwasser folgender Zusammensetzung geprüft:

*Lösung A*

55,8 g NaCl, 25,4 g $MgCl_2 \cdot 6H_2O$, 2,6 g $CaCl_2$, 1,6 g KCL in 1 000 g destilliertem Wasser.

*Lösung B*

21,2 g $Na_2SO_4 \cdot 10 H_2O$ in 117 g destilliertem Wasser.

Die Lösung B wird unter Rühren der Lösung A zugegeben und nach einem Tag Standzeit wird die Prüfflüssigkeit durch Blaubandfilter abfiltriert. Die Prüfung des Flüssigkeitsaufnahmevermögens erfolgt durch Tauchen des pulverförmigen Polymeren, das auf einer Glasfritte aufgestreut ist, in die Prüfflüssigkeit. Danach wird das aufgequollene Polymergel bei 85°C 6 bis 8 h getrocknet (der 1. Zyklus) und die Prüfflüssigkeitsaufnahme nach gleicher Methode wiederholt (der 2. und weitere Zyklen). Die Ergebnisse sind in folgender Tabelle zusammengefasst, wobei gleichzeitig auch die Vergleichswerte, die mit den dem Stand der Technik entsprechenden Produkten erreicht wurden, angegeben sind.

*Beispiel 9*

In einem Polymerisationsgefäss wurden 332 g Acrylamid und 108 g Acrylamidomethylpropansulfonsäure in 1 000 g Wasser gelöst und mit 36 g Kaliumcarbonat neutralisiert. Danach wurde 3,5 g Tetraallyloxyethan zugegeben und mit 0,73 g Azobisamidinpropandihydrochlorid katalysiert. Die Reaktion verlief praktisch adiabatisch, wobei die Temperatur von 15 bis zu 86°C gestiegen ist. Das entstandene Polymergel wurde zerkleinert, gegtrocknet und gemahlen.

Das Produkt wurde mit einem künstlichen Meerwasser wie im Beispiel 8 geprüft.

*Tabelle*

|  | Prüfflüssigkeitsaufnahme (g/ml) im | | | | |
|---|---|---|---|---|---|
|  | 1. Zyklus | 3. Zyklus | 5. Zyklus | 7. Zyklus | 9. Zyklus |
| Copolymerisat gemäss Beispiel 8 | 17,0 | 13 | 12,5 | 14,0 | 13 |
| Copolymerisat gemäss Beispiel 9 | 22,0 | 17,5 | 16,9 | 18,8 | 17,3 |
| Vergleichsbeispiel vernetzte Polyacrylsäure | 17,0 | 9,7 | 5,4 | 4,2 | 3,6 |
| Vergleichsbeispiel vernetztes Acrylamid/ Acrylsäure-Copolymerisat 90/10 | 16,0 | 3,5 | 2,7 | 1,5 | 1,0 |

Die erfindungsgemässen Produkte absorbieren nach 9 Zyklen noch 13 bis 17 ml/g Meerwasser im Vergleich zu den vernetzten Acrylamid/Acrylsäure-Copolymerisaten oder dem Acrylsäurehomopolymerisat, die im 9. Zyklus nur noch eine Absorption von 1,0 bis 3,6 ml/g aufweisen.

## Patentansprüche

1. Vernetzte, in Wasser quellbare Copolymerisate, bestehend aus

A) 0,5 bis 75, vorzugsweise 5 bis 30 Gew.-% Grundeinheiten, die sich von 2-Acrylamido-2-methylpropansulfonsäure oder deren Alkali- und/oder Ammoniumsalzen ableiten,

B) 23 bis 99,49, vorzugsweise 70 bis 95 Gew.-% Grundeinheiten, die sich von Acrylsäure und/oder Methacrylsäure oder deren Alkali- und/oder Ammoniumsalzen und/oder Acrylamid und/oder Vinylpyrrolidon ableiten, und

C) 0,01 bis 2,0 Gew.-% an Grundeinheiten, die sich von einem mindestens bifunktionellen Vernetzer ableiten.

2. Absorptionsmittel für Körperflüssigkeiten, bevorzugt Urin und andere elektrolythaltige wässerige Lösungen, gekennzeichnet durch einen Gehalt an Copolymerisaten nach Anspruch 1, gegebenenfalls zusammen mit weiteren hydrophilen Absorptionsmitteln in einer Menge von 0,1 bis 10, vorzugsweise 0,2 bis 5,0 Gew.-%, bezogen auf Copolymerisat.

3. Absorptionsmittel nach Anspruch 2, gekennzeichnet durch einen Gehalt an entweder auf pyrolytischem Wege oder durch Fällung aus Wasserglaslösungen gewonnener feinverteilter Kieselsäure als weiteres hydrophiles Absorptionsmittel.

4. Verwendung der Copolymerisate nach Anspruch 1 und des Absorptionsmittels nach den Ansprüchen 2 und 3 zur Aufnahme und/oder Zurückhaltung von Urin oder anderen elektrolythaltigen wässerigen Lösungen, insbesondere in Saugkörpern auf Textil- oder Papierbasis.

## Claims

1. Cross-linked copolymers that are capable of swelling in water, comprising—

(A) from 0.5 to 75% by weight, preferably from 5 to 30% by weight, of structural units that are derived from 2-acrylamido-2-methylpropanesulphonic acid or the alkali metal and/or ammonium salts thereof,

(B) from 23 to 99.49% by weight, preferably from 70 to 95% by weight, of structural units that are derived from acrylic acid and/or methacrylic acid or the alkalimetal and/or ammonium salts thereof and/or acrylamide and/or vinyl-pyrrolidone, and

(C) from 0.01 to 2.0% by weight of structural units that are derived from an at least bifunctional crosslinking agent.

2. Absorbent for body fluids, preferably urine and other aqueous solutions containing electrolytes, characterised by a content of copolymers according to claim 1, optionally together with additional hydrophilic absorbents in an amount of from 0.1 to 10, preferably 0.2 to 5.0% by weight, based on the copolymer.

3. Absorbent according to claim 2, characterised by a content of finely divided silica obtained either by pyrolysis or by precipitation from water glass solutions as additional hydrophilic absorbent.

4. Use of the copolymers according to claim 1 and of the absorbent according to claims 2 and 3 for absorption and/or retention of urine or other aqueous solutions containing electrolytes, especially in absorbent articles based on textiles or paper.

## Revendications

1. Copolymères réticulés, gonflables dans l'eau, constitués de:

A) 0,5 à 75 et de préférence 5 à 30% en poids d'unités de base qui dérivent de l'acide acryl-amido-2 méthyl-2 propanesulfonique ou des sels de métaux alcalins et/ou d'ammonium de celui-ci,

B) 23 à 99,49 et de préférence 70 à 95% en poids d'unités de base qui dérivent de l'acide acrylique et/ou de l'acide méthacrylique ou de sels de métaux alcalins et/ou d'ammonium de ces acides et/ou de l'amide acrylique et/ou de la vinyl-pyrrolidone,

C) 0,01 à 2,0% en poids d'unités de base qui dérivent d'un agent de réticulation au moins difonctionnel.

2. Produit absorbant pour des liquides corporels tels qu'en particulier l'urine et d'autres solutions aqueuses contenant des électrolytes, caractérisé en ce qu'il contient des copolymères suivant la revendication 1, ainsi qu'éventuellement entre 1 et 10 et de préférence entre 0,2 et 5,0% en poids, par rapport au copolymère, d'autres substances absorbantes hydrophiles.

3. Produit absorbant suivant la revendication 2, caractérisé en ce qu'il contient comme substance absorbante hydrophile additionnelle de l'acide silicique finement divisé, préparé par voie pyrolytique ou par précipitation à partir de solutions de verre soluble.

4. Utilisation des copolymères suivant la revendication 1 et du produit absorbant suivant les revendications 2 et 3 pour l'absorption et/ou la rétention d'urine ou d'autres solutions aqueuses contenant des électrolytes, en particulier sous forme d'articles absorbants à base de textile ou de papier.